# EUROPÄISCHE PATENTSCHRIFT

(11) **EP 1 102 622 B1**
(45) Veröffentlichungstag und Bekanntmachung des Hinweises auf die Patenterteilung: **05.11.2003**
(21) Anmeldenummer: 99932777.8
(22) Anmeldetag: 01.07.1999
(51) Int. Cl.: B01D 63/16, B01D 65/08

(54) **FILTRIERVORRICHTUNG**
FILTRATION DEVICE
DISPOSITIF DE FILTRATION

(30) Priorität: 03.08.1998 DE 19834915; 13.11.1998 DE 19852466
(43) Veröffentlichungstag der Anmeldung: 30.05.2001
(73) Patentinhaber: PLASMASELECT AKTIENGESELLSCHAFT, 17166 Teterow (DE)
(72) Erfinder: SCHIMMELPFENNIG, Winfried, D-18292 Krakow (DE); LANTOW, Holger, D-18146 Rostock (DE); MÜLLER, Uwe, D-90449 Nürnberg (DE); RIGGERS, Wilfried, D-27432 Bremerförde (DE)
(74) Vertreter: Grünecker, Kinkeldey, Stockmair & Schwanhäusser Anwaltssozietät
(86) Internationale Anmeldenummer: EP9904554
(87) Internationale Veröffentlichungsnummer: WO00007701

(56) Entgegenhaltungen:
- EP-A- 0 238 335
- WO-A-90/00069
- WO-A-97/19745
- PATENT ABSTRACTS OF JAPAN vol. 013, no. 422 (P-933), 20. September 1989 (1989-09-20) & JP 01 155266 A (TORAY IND INC), 19. Juni 1989 (1989-06-19)

## Beschreibung

Die vorliegende Erfindung betrifft eine Filtriervorrichtung gemäß dem Oberbegriff von Anspruch 1. Eine derartige Vorrichtung ist aus der EP-A-0 238 335 bekannt.

Eine ähliche Vorrichtung ist aus der US-A-5,034,135 bekannt. Bei diesem vorbekannten Stand der Technik wird eine zu filtrierende Flüssigkeit in einen zylinderförmigen Ringspalt eingeleitet, der sich zwischen einem ortsfesten äußeren Zylinder, der die Gegenfläche bildet, und einem rotierenden Filterzylinder, an dem die Filtermembran angeordnet ist, erstreckt. Diese Vorrichtung eignet sich insbesondere zur Filtration von Blut, um die Bestandteile Blutplasma einerseits und die Blutzellen andererseits voneinander zu trennen. Die US-A-5,034,135 lehrt, daß aufgrund des rotierenden Filterzylinders eine nach außen gerichtete Zentrifugalkraft erzeugt wird, die die Blutzellen von der Membran nach außen treibt, während gleichzeitig aufgrund eines über die Filtermembran wirkenden Transmembrandruckes das Blutplasma von den Blutzellen getrennt wird. Das derart erzeugte Filtrat (Blutplasma) wird in dem Filter in radialer Richtung nach innen geführt und über eine Dreh-Dichtung, die in dem Drehpunkt des Filterzylinders angeordnet ist, kontinuierlich nach außen geleitet.

Die vorbekannte Vorrichtung ist üblichen Filtervorrichtungen, bei denen eine Suspension oder aber eine Emulsion an einer mikroporösen Membran ausschließlich aufgrund des Transmembrandruckes in Filtrat einerseits und Konzentrat andererseits aufgespalten wird, überlegen, da durch die Drehung des Filterzylinders eine Relativbewegung der Flüssigkeit zu der Filtermembran erzeugt wird und somit ein Verstopfen der Filterporen durch die Festkörper bzw. Zellen oder emulgierte Tröpfchen, verhindert wird. Nachteilig bei der vorbekannten Vorrichtung ist jedoch, daß sie relativ voluminös ist. Darüber hinaus erfordert der Aufbau der vorbekannten Vorrichtung einen relativ hohen konstruktiven Aufwand. Dies gilt insbesondere für die Dreh-Dichtung, die im übrigen auch zu einer Leckage führen kann. Insbesondere beim Filtrieren von Blut ergibt sich weiterhin das Problem einer sicheren und zuverlässigen Abdichtung.

Aus der WO 97/19745 ist eine Vorrichtung mit einem rotierenden Drehkörper bekannt, welcher zwischen Leitblechen angeordnet ist, die an ihrem radial äußeren Ende über eine sich im Wesentlichen parallel zu der Drehachse des Drehkörpers erstreckende Filtermembran verbunden sind. Der Drehkörper weist an seiner Oberfläche spiralförmig ausgebildete Stege auf, die bei Drehung des Drehkörpers eine Strömung der zu filtrierenden Flüssigkeit vom Mittelpunkt der Drehachse radial nach außen zu und schließlich durch die Filtermembran erzwingt. Die zu filtrierende Flüssigkeit ist in einem Behälter aufgenommen, in den auch der Drehkörper und die Filtermembran eingetaucht sind. Diese vorbekannte Filtriervorrichtung ist sehr aufwendig und aufgrund der relativ geringen Filteroberfläche verhältnismäßig uneffizient.

Aus der WO 90/00069 ist eine Behandlungseinrichtung für menschliches Blut umfassend eine Filtriereinrichtung vorbekannt, bei der zunächst die korpuskulären Bestandteile von Blut von den flüssigen Bestandteilen mittels Filtration getrennt, die konzentrierten Blutzellen gewaschen und nachfolgend die gewaschenen Blutzellen in eine zweite Filtrationszone verbracht werden, um diese wiederum von der Waschflüssigkeit zu trennen. Die Ausbildung unterschiedlicher Behandlungszonen führt zu einem sehr aufwendigen Aufbau. Die vorbekannte Vorrichtung ist daher verhältnismäßig teuer und unwirtschaftlich im Einsatz, wenn es lediglich darum geht, Flüssigkeit, insbesondere Blut zu filtrieren.

Auch die aus dem eingangs genannten gattungsbildenden Stand der Technik (EP-A-0 238 335) vorbekannte Filtriervorrichtung ist verhältnismäßig aufwendig aufgebaut und weist drei unterschiedliche, jeweils eine Flüssigkeitskammer bildende Bauteile auf. Die Flüssigkeitskammern sind jeweils über eine Filtriermembran voneinander getrennt. Auch wird die Strömung der zu filtrierenden Flüssigkeit innerhalb der Vorrichtung dadurch behindert, daß die Strömungsein- und auslässe mit geringem Abstand zu dem Drehkörper ausgebildet sind.

Der vorliegenden Erfindung liegt die Aufgabe zugrunde, eine Filtriervorrichtung der eingangs genannten Art weiterzubilden, die einen geringen Platzbedarf hat und die sich insbesondere einfacher und kostengünstiger herstellen lässt.

Zur Lösung der obigen Aufgabe wird mit der vorliegenden Erfindung eine Filtriervorrichtung mit den Merkmalen von Anspruch 1 angegeben. Bevorzugte Weiterbildungen sind in den Unteransprüchen angegeben.

Da der Flüssigkeitsraum sich parallel zu der Fläche eines Drehkörpers erstreckt, die im wesentlichen senkrecht zu der Drehachse des Drehkörpers angeordnet ist, läßt sich die erfindungsgemäße Filtriervorrichtung bei relativ großer Oberfläche im Vergleich zu der vorbekannten Trommelanordnung verhältnismäßig platzsparend verwirklichen. Durch die ortsfeste Anordnung der Membranen ist der konstruktive Aufbau der erfindungsgemäßen Filtriervorrichtung vereinfacht. Über den Ringkanal kann die Flüssigkeit gesammelt und zu dem Flüssigkeitsauslaß geleitet werden. Aufgrund der Ausbildung von Flüssigkeitseinlaß und -auslaß an unterschiedlichen Gehäusehälften kann das Gehäuse vorzugsweise aus zwei identisch ausgebildeten Gehäusehälften geformt werden, was im Hinblick auf eine vereinfachte Herstellung der erfindungsgemäßen Vorrichtung die zu bevorzugende Ausführungsform der Erfindung darstellt.

Der Drehkörper der erfindungsgemäßen Filtriervorrichtung weist eine wesentlich größere radiale als axiale Erstreckung auf und wird vorzugsweise durch eine Scheibe, insbesondere eine Kreisscheibe gebildet. Die Oberfläche kann eben oder aber strukturiert ausgebildet sein, um die Ausformung von Mikrowirbeln zu begünstigen. im Hinblick auf eine kostengünstige und einfache Herstellung ist es zu bevorzugen, den Drehkörper als ebene Scheibe auszubilden. Alternativ kann die Scheibe aber auch beispielsweise einen kegelförmigen oder kegelstumpfförmigen Querschnitt aufweisen.

Bei einer weiteren bevorzugten Ausgestaltung der vorliegenden Erfindung, die sich insbesondere für die Filtration von toxischen Stoffen oder aber von Blut oder anderen Körperflüssigkeiten eignet, ist der Drehkörper berührungsfrei antreibbar. Dementsprechend kann auf eine das Gehäuse der Filtriervorrichtung durchdringende, verdrehfest mit der Scheibe verbundene Welle verzichtet werden. Das Gehäuse ist vielmehr im Bereich der Lagerung der Welle nach außen abgedichtet, so daß ein Austreten, beispielsweise von toxischer Flüssigkeit oder ein Eindringen von Keimen oder Bakterien in den Flüssigkeitsraum sicher vermieden wird. Besonders zu bevorzugen ist für einen berührungsfreien Antrieb ein Drehkörper, der metallisch leitend ist, so daß der Antrieb über magnetische Induktion mittels eines von außen eingebrachten rotierenden Magnetfeldes bewirkt wird. Ein Wechsel verbrauchter Filtriervorrichtungen und eine Kopplung mit dem Antrieb für den drehbar gelagerten Drehkörper läßt sich somit auf einfache Weise durchführen.

Weitere Einzelheiten, Merkmale und Vorteile der vorliegenden Erfindung ergeben sich aus der nachfolgenden Beschreibung von Ausführungsbeispielen der vorliegenden Erfindung in Verbindung mit der beigefügten Zeichnung. In dieser zeigen:
- Fig. 1: eine schematische Darstellung der in dem spaltförmigen Flüssigkeitsraum erzeugten Makroströmung;
- Fig. 2: eine Längsschnittansicht eines Ausführungsbeispiels der vorliegenden Erfindung und
- Fig. 3: eine Schnittansicht entlang der Linie III-III gemäß Fig. 2.

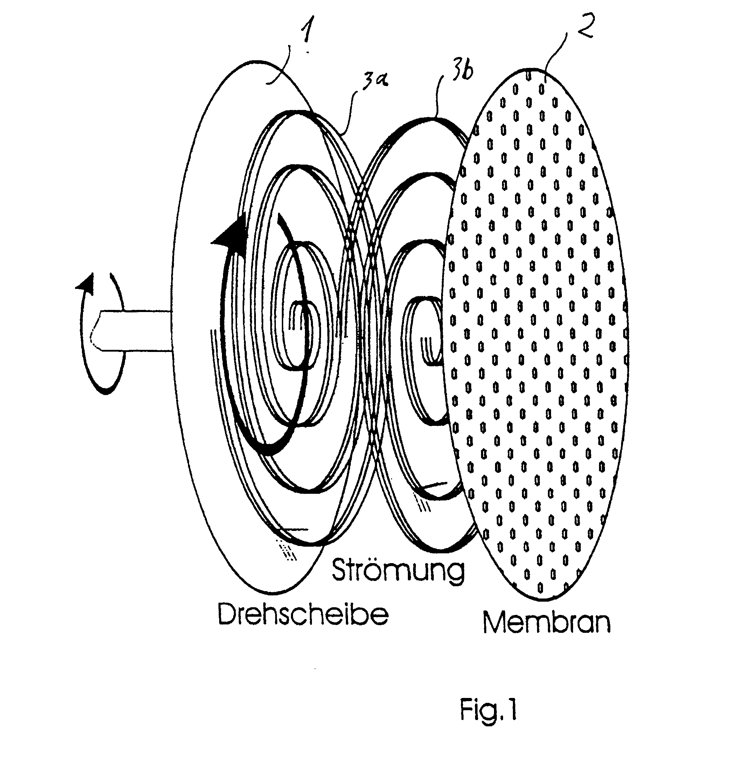

Fig. 1 zeigt eine schematische Ansicht von wesentlichen Teilen eines Ausführungsbeispiels der vorliegenden Erfindung, das eine drehende Scheibe 1 und eine ortsfeste Membran 2 aufweist. Die sich im stationären Zustand, d.h. bei konstanter Drehung der Scheibe ergebende Strömung umfaßt einerseits einen durch die Drehung der Scheibe 1 bewirkten spiralförmigen Flüssigkeitswirbel 3a, der sich parallel zu der Kreisfläche der Scheibe 1 erstreckt und benachbart zu der Scheibe 1 ausgebildet ist. Benachbart zu der Membran 2 bildet sich ebenfalls aufgrund der durch die Drehung der Scheibe 1 erzeugten Scherkräfte ein Wirbel 3b aus. Der Wirbel 3a und der Wirbel 3b haben den gleichen Drehsinn. Der benachbart zu der Scheibe 1 ausgebildete spiralförmige Flüssigkeitswirbel 3a hat jedoch eine nach außen gerichtete radiale Geschwindigkeitskomponente, wohingegen der benachbart zu der Membran 2 angeordnete spiralförmige Flüssigkeitswirbel 3b eine radial nach innen gerichtete Geschwindigkeitskomponente hat.

Die in einen zwischen der Scheibe 1 und der Membran 2 angeordneten spiralförmigen Flüssigkeitsraum 12 eingeleitete Flüssigkeit bewegt sich zunächst über den Flüssigkeitswirbel 3b entlang der Membran 2 und im Drehsinn der Scheibe 1 radial nach innen, bis sie ungefähr den Mittelpunkt der Scheibe 1 erreicht und wird dann in den zweiten spiralförmigen Flüssigkeitswirbel 3a überführt, der das Konzentrat in einer spiralförmigen Bewegung parallel zur Scheibe 1 radial nach außen fördert. Mikrowirbel, die insbesondere in der Nähe der Membran 2 erzeugt werden, um ein Verstopfen derselben zu verhindem, lassen sich insbesondere durch Einstellen des Abstandes zwischen der Scheibe 1 und der Membran 2 und durch Einstellen der Drehzahl der Scheibe 1 beeinflussen. Die durch die Drehung der Scheibe 1 entstehende Zentrifugalkraft in der rotierenden Flüssigkeit trägt nicht zur Separation der in der Flüssigkeit enthaltenen Partikel bei, da die Zentrifugalkraft lediglich parallel zur Oberfläche der Membran 2 bzw. der Scheibe 1 wirkt. Die Ausbildung der Makrowirbel in dem spaltförmigen Flüssigkeitsraum wird von zwei wesentlichen Größen beeinflußt. Dies ist zum einen die Scherung zwischen der angetriebenen Oberfläche der Scheibe 1 und der vorliegend ortsfesten Oberfläche der Membran 2, durch die die Flüssigkeiten tangentialer Richtung bewegt wird. Zum anderen treibt die Zentrifugalkraft die Flüssigkeit aus dem Spalt. Da diese jedoch in der Nähe der rotierenden Scheibe größer ist als in der Nähe der ortsfesten Membran, ergibt sich aufgrund der Überlagerung von Scherkraft einerseits und Zentrifugalkraft andererseits ein gerichtetes Abfördern von Konzentrat über den spiralförmigen Flüssigkeitswirbel 3a, wohingegen über den Gegenwirbel 3b aufgrund der Volumenkonstanz Flüssigkeit eingezogen wird.

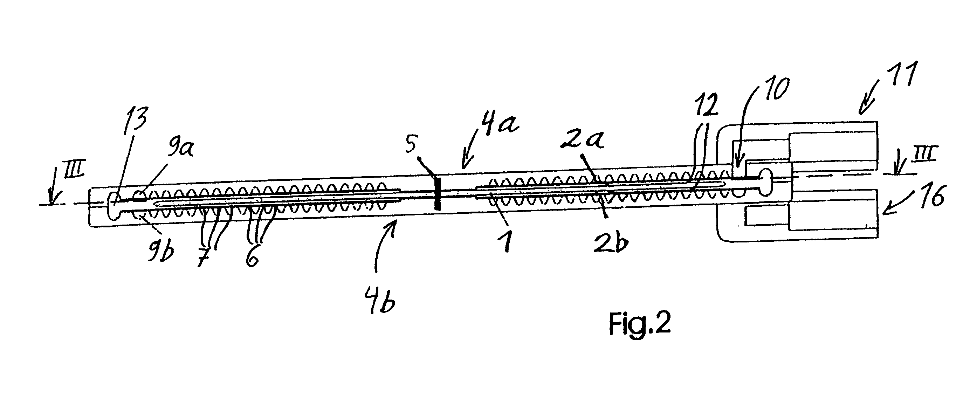

In Fig. 2 ist eine Längsschnittansicht einer Filtriervorrichtung für Blut als ein weiteres Ausführungsbeispiel der vorliegenden Erfindung gezeigt, die sich zur Plasmapherese eignet. Das Ausführungsbeispiel umfaßt zwei kreisförmig ausgebildete, flache Halbschalen 4a, 4b, die jeweils eine Membran 2 tragen. Zwischen den parallel zueinander angeordneten Membranen 4a, 4b ist eine Scheibe 1 angeordnet, die über einen Lagerstift 5 drehbar gelagert ist. Auf der der Scheibe 1 abgewandten Seite der jeweiligen Membranen 2a, 2b sind in radialer Richtung abwechselnd Membranhaltestege 6 und Filtratkanäle 7 angeordnet, wobei die Membranhaltestege 6 ringsegmentförmig an den Halbschalen 4 einstückig mittels Spritzgießen ausgebildet sind. Die Halbschalen 4 bestehen vorzugsweise aus einem transparenten Acrylwerkstoff.

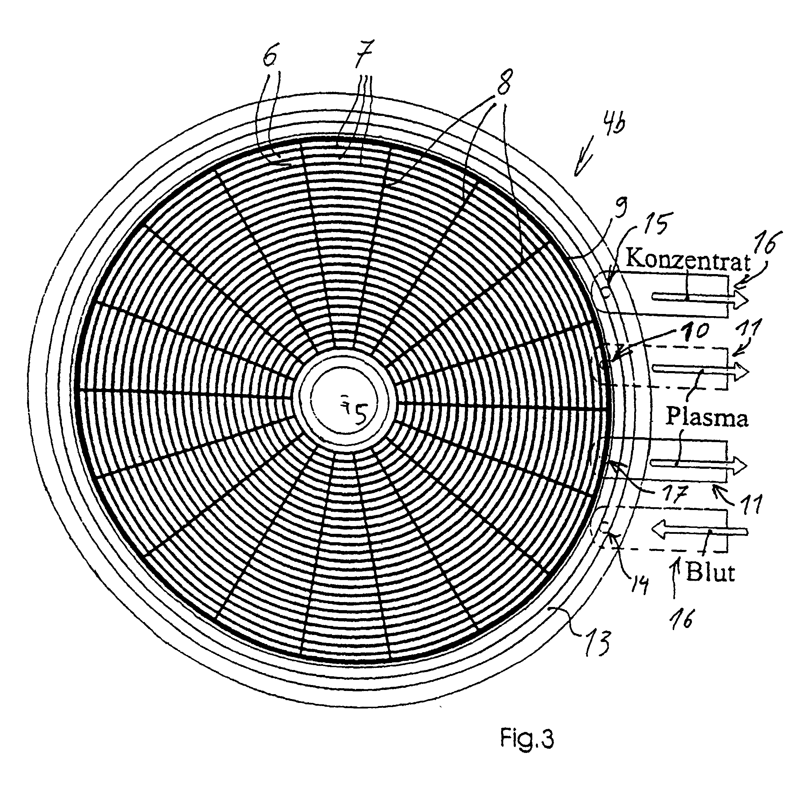

Wie den Schnittansichten gemäß den Fig. 2 und 3 zu entnehmen ist, verlaufen die Filtratkanäle 7 konzentrisch zu dem Lagerstift 5 der Scheibe 2. In Umfangsrichtung sind eine Vielzahl von, sich in radialer Richtung erstreckende Filtratabführkanäle 8 vorgesehen, die in einen ringförmig um die Scheibe 2 angeordneten Filtratringkanal 9 münden. Der Filtratkanal 9 wiederum weist einen Filtratauslaß 10, der an der einen Gehäusehälfte 4a zu dem an dieser ausgebildeten Filtratringkanal 9a vorgesehen ist, und einen Filtratauslaß 17 auf, der in identischer Weise an der anderen Gehäusehälfte 4b zu dem an dieser ausgebildeten Filtratringkanal 9b vorgesehen ist, auf. Beide Filtratauslässe 10, 17 kommunizieren mit Schlauchanschlüssen 11.

Zwischen den jeweiligen Membranen 2a, 2b und der Scheibe 1 ist jeweils ein spaltförmiger Flüssigkeitsraum 12a, 12b ausgebildet. Jeder spaltförmige Flüssigkeitsraum 12 kommuniziert mit einem diesen radial umgebenden und mit diesem kommunizierenden Flüssigkeitsringkanal 13. Der Flüssigkeitsringkanal 13 weist einen Flüssigkeitseinlaß 14 und einen Konzentratauslaß 15 auf. Auch diese Strömungsdurchlässe 14, 15 sind mit Schlauchanschlüssen 16 verbunden.

Bei dem gezeigten Ausführungsbeispiel weist der Flüssigkeitsringkanal 13 einen größeren Strömungsquerschnitt auf als der zwischen den beiden Membranen 2a, 2b gebildete Spalt 12. Der Flüssigkeitsringkanal 13 ist kreisförmig ausgebildet und konzentrisch zu dem Lagerstift angeordnet, wobei der Durchmesser des Flüssigkeitsringkanals 13 größer ist, als der Durchmesser des Filtratringkanales 9.

Bei dem gezeigten Ausführungsbeispiel werden die Schlauchanschlüsse 11, 16 mit Schläuchen verbunden, wobei der mit dem Flüssigkeitseinlaß verbundene Schlauch beispielsweise an einen Spender angeschlossen ist. Durch den Flüssigkeitseinlaß 14 in die beiden spaltförmigen Flüssigkeitsräume 12a, 12b eingeleitetes Blut wird zunächst aufgrund der Überlagerung von Scherung und Zentrifugalkraft benachbart zu der Membran und parallel zu dieser in einem spiralförmigen, in jedem der spaltförmigen Flüssigkeitsräume 12a, 12b ausgebildeten Flüssigkeitswirbel nach innen geleitet. Aufgrund der Scherung erzeugte Mikrowirbel sorgen dafür, daß die Poren der Membranen 2a, 2b nicht durch insbesondere Blutkörperchen verstopft werden. Auf dem Weg entlang der Membran radial nach innen erfolgt die Filtration des in die Filtriervorrichtung eingeleiteten Blutes, und zwar in herkömmlicher Weise aufgrund einer Druckdifferenz, die über der Membran anliegt. Das derart erzeugte Filtrat wird über die zwischen den Membranhaltestegen 6 ausgebildeten Filtratkanäle 7 in die Filtratabführkanäle 8 geleitet und über diese in dem Filtratringkanal 9 gesammelt.

Das durch den Flüssigkeitseinlaß 14 in den Flüssigkeitsringkanal 13 eingeleitete Blut wird aufgrund der Drehbewegung der beiden in dem jeweiligen spaltförmigen Flüssigkeitsraum 12a; 12b zwischen den jeweiligen Membranen 2a; 2b und der Scheibe 1 ausgebildeten spiralförmigen Flüssigkeitswirbel mit nach innen gerichteter radialer Bewegungskomponente mitgenommen.

Das in den Fig. 2 und 3 gezeigte Ausführungsbeispiel weist den besonderen Vorteil auf, daß es als Einwegartikel nutzbar ist. Die beiden Gehäusehälften 4a, 4b sind in identischer Weise ausgebildet, wobei an jeder Gehäusehälfte 4a, 4b im vorliegenden Fall ein von der äußeren Stirnseite der Gehäusehälfte vorspringender Schlauchanschluß 11 zur Ableitung des Filtrates (Plasma) vorgesehen ist. An jeder Gehäusehälfte 4a, 4b ist außerdem ein weiterer Schlauchanschluß 16 vorgesehen, der bei der einen Gehäusehälfte 4b für die Ableitung des Konzentrates verwendet wird und mit dem Konzentratauslaß 15 kommuniziert und bei der anderen Gehäusehälfte 4b für die Zufuhr des unfiltrierten Blutes verwendet wird und mit dem Flüssigkeitseinlaß 14 kommuniziert. Durch diesen Aufbau ist es möglich, das Ausführungsbeispiel zur Filtration von Blut als Einwegteil bestehend aus im wesentlichen zwei identisch ausgeformten, spritzgegossenen Gehäusehälften 4a, 4b, zwei Membranen 2a, 2b und einer Scheibe 1 mit Lagerzapfen mit Lagerzapfen 5 herzustellen.

Eine konkrete Ausgestaltung der Filtriervorrichtung für Blut könnte beispielsweise eine aus V2A-Stahl gefertigte Drehscheibe aufweisen, die 0,8 mm dick ist. Die Filtratabführkanäle 8 können vorzugsweise mit einem Abstand von 20° in Umfangsrichtung verteilt angeordnet sein, um eine ganzflächige Abförderung des Filtrates, d.h. des separierten Blutplasmas zu ermöglichen. Für eine Blutfiltriervorrichtung werden vorzugsweise Membranen aus Cellulose-Acetat mit einer Porengröße von ca. 1 µm verwendet. Diese haben vorzugsweise einen Durchmesser von 10 cm und sind ringförmig an dem Außenrand des jeweiligen Filtratkanals befestigt. Die Befestigung kann beispielsweise über eine Klebeoder Schweißverbindung erfolgen. Auch die beiden Gehäusehälften sind miteinander verbunden, wobei auch diese Verbindung mit einer Schweiß- oder Klebeverbindung verwirklicht werden kann. Alternativ ist es auch möglich, die beiden Gehäusehälften über eine Rastverbindung miteinander zu verbinden, so daß sich die im wesentlichen identisch ausgebildeten Gehäusehälften lediglich durch männliche und weibliche Rastelemente unterscheiden. Wesentlich für die Verbindung der beiden Gehäusehälften ist jedoch, daß diese flüssigkeitsdicht miteinander verbunden sind. So können beispielsweise die beiden Gehäusehälften unter Zwischenlage einer ringförmigen Dichtung miteinander in ihrem Randbereich verbunden sein.

Die gezeigte Filtriervorrichtung ist ungefähr 6 mm dick, wobei jede Gehäusehälfte ca. 2 mm dick ist und die Filtratabführkanäle 8 sowie die Filtratkanäle 7 eine Tiefe von ungefähr 1 mm aufweisen. Die Membranen 2a, 2b sind jeweils ungefähr 0,1 mm dick, wobei der Abstand zwischen den Membranen 2a, 2b und der Scheibe 1 zwischen 0,4 und 0,6 mm, beispielsweise 0,5 mm beträgt.

Bei einem gemäß den vorstehend genannten Zahlenangaben demissionierten Ausführungsbeispiel beträgt das Füllvolumen für die Filtriervorrichtung ca. 10 ml. Die Scheibe wird bei der Filtration von Blut in diesem Fall mit einer Geschwindigkeit von 600 bis 1.400, vorzugsweise 800 bis 1.200 Umdrehungen pro Minute gedreht. Bei einer derartigen Geschwindigkeit wird eine im Hinblick auf die Blutschädigung ausreichend geringe Schergeschwindigkeit von ca. 10.000 1/s erzeugt. Der Transmembrandruck wird auf ungefähr 200 mbar bis 300 mbar, vorzugsweise 225 mbar bis 275 mbar eingestellt. Bei diesen Prozeßparametem kann bei einer Behandlungszeit von 4 Stunden im Durchschnitt ein Ertrag von über 8 Liter Plasma bei kontinuierlicher Zufuhr von Blut mit einer Fließgeschwindigkeit von 100 ml pro Minute erzielt werden.

Die Auslegung der erfindungsgemäßen Vorrichtung wie Materialien, Scheibengröße, Oberflächenbeschaffenheit, Membranfläche, Abstand von Membran- und Scheibenoberfläche, Drehzahl der Scheibe und Transmembrandruck hängen wesentlich von den Eigenschaften der zu trennenden Medien ab. Die erfindungsgemäße Filtriervorrichtung ist nicht auf die Filtration von Blut beschränkt. Im übrigen sollte Filtration im Sinne der vorliegenden Erfindung nicht ausschließlich als Vorgang verstanden werden, bei dem aus einer Suspension feste Partikel entfernt werden. Vielmehr läßt sich die erfindungsgemäße Filtriervorrichtung auch anwenden, um beispielsweise Emulsionen zu separieren.

## Patentansprüche

1. Filtriervorrichtung mit zwei Filtermembranen (2) und den Filtermembranen (2) gegenüberliegend ausgebildeten Gegenflächen und jeweils einem zwischen der Filtermembran und der Gegenfläche ausgebildeten spaltförmigen Flüssigkeitsraum (12), wobei die Gegenflächen als gegenüberliegende Flächen eines Drehkörpers (1) ausgebildet und im Wesentlichen senkrecht zu der Drehachse des Drehköpers (1) angeordnet sind,
**dadurch gekennzeichnet,**
**dass** die Filtriervorrichtung zwei als Spritzgießteile ausgebildete Gehäusehälften (4a, 4b) aufweist, zwischen denen ein den Drehkörper (1) umgebender Ringkanal (13) ausgebildet ist, der mit einem Flüssigkeitseinlass (14) und einem Flüssigkeitsauslass (15) versehen ist, die jeweils mit einem Schlauchanschluss kommunizieren und die an unterschiedlichen Gehäusehälften (4a, 4b) vorgesehen sind.

2. Filtriervorrichtung nach Anspruch 1, **dadurch gekennzeichnet, dass** jede Gehäusehälfte (4a, 4b) jeweils zwei Schlauchanschlüsse (11, 16) aufweist.

3. Vorrichtung nach einem der vorherigen Ansprüche, **dadurch gekennzeichnet, dass** der Drehkörper (1) eine wesentlich größere radiale als axiale Erstreckung aufweist.

4. Vorrichtung nach einem der vorherigen Ansprüche, **dadurch gekennzeichnet, dass** die Fläche eben und rechtwinkelig zu der Drehachse des Drehkörpers (1) ausgebildet ist.

5. Filtriervorrichtung nach zumindest einem der vorherigen Ansprüche, **dadurch gekennzeichnet, dass** der Drehkörper (1) berührungsfrei antreibbar ist.

6. Filtriervorrichtung nach Anspruch 5, **dadurch gekennzeichnet, dass** der Drehkörper (1) metallisch leitend und/oder magnetisierbar ist.

7. Filtriervorrichtung nach zumindest einem der vorherigen Ansprüche, **dadurch gekennzeichnet, dass** an der dem Flüssigkeitsraum abgewandten Seite der Filtermembran (2) Membranhaltestege (6) vorgesehen sind, durch die die Membran (2) derart abgestützt ist, dass die spaltförmige Form des Flüssigkeitsraumes auch bei einer mechanischen Beanspruchung der Filtermembran (2) erhalten bleibt.

8. Filtriervorrichtung nach zumindest einem der vorherigen Ansprüche, **gekennzeichnet durch** zwei den Drehkörper (1) zwischen sich einschließende Gehäusehälften (4a, 4b), die derart ausgestaltet sind, dass identische spaltförmige Flüssigkeitsräume (12) beiderseits des Drehkörpers (1) ausgebildet sind.

## Claims

1. A filtering device comprising two filtering membranes (2) and counter surfaces opposite to the filtering membrane and one gap-shaped liquid space (12) formed between the filtering membrane and the counter surface, wherein the counter surfaces are formed as opposing surfaces of a rotary body which are arranged substantially perpendicularly to the axis of rotation of the rotary body (1)
**characterized in that**
the filtering device has to housing halves (4a, 4b) formed as injection molding members, between which said halves an annular channel (13) surrounding the rotary body (1) is formed, said annular channel being provided with a liquid inlet (14) and a liquid outlet (15) each communicating with a hose connection and being provided on different housing halves (4a, 4b).

2. A filtering device as claimed in claim 1, **characterized in that** each housing half (4a, 4b) each comprises two hose connections (11, 16).

3. A device as claimed in one of the preceding claims, **characterized in that** the rotary body (1) has a substantially larger radial than axial extension.

4. A device as claimed in one of the preceding claims, **characterized in that** the surface is formed planarly and at a right angle with respect to the axis of rotation of the rotary body (1).

5. A filtering device as claimed in at least one of the preceding claims, **characterized in that** the rotary body (1) can be driven in a contact-free manner.

6. A filtering device as claimed in claim 5, **characterized in that** the rotary body (1) is metallically conductive or magnetizeable.

7. A filtering device as claimed in at least one of the preceding claims, **characterized in that** membrane supporting webs (6) are provided at the side of the filtering membrane (2) opposite to the liquid space, said membrane supporting webs supporting said membrane (2) in such a manner that the gap-like shape of the liquid space is also maintained in case of a mechanical bad of the filtering membrane (2).

8. A filtering device as claimed in at least one of the preceding claims, **characterized by** two housing halves (4a, 4b) between which the rotary body (1) is arranged, said housing halves being designed in a manner that identical gap-shaped liquid spaces (12) are formed an both sides of the rotary body (1).

## Revendications

1. Dispositif de filtration comportant deux membranes filtrantes (2) et des surfaces conjuguées aménagées en vis-à-vis des membranes filtrantes (2) et une chambre de liquide (12) en forme d'interstice située entre chaque membrane filtrante et chaque surface conjuguée, les surfaces conjuguées étant des surfaces en vis-à-vis d'un corps tournant (1) et étant disposées pour l'essentiel perpendiculairement à l'axe de rotation du corps tournant (1),
**caractérisé en ce**
**que** le dispositif de filtration présente deux moitiés de boîtier (4a, 4b) qui sont réalisées sous forme de pièces moulées par injection et entre lesquelles se trouve un canal annulaire (13) qui entoure le corps tournant (1) et est pourvu d'une entrée de liquide (14) et d'une sortie de liquide (15) qui communiquent chacune avec un raccord de tuyau et sont prévues sur des moitiés de boîtier (4a, 4b) différentes.

2. Dispositif de filtration selon la revendication 1, **caractérisé en ce que** chaque moitié de boîtier (4a, 4b) comporte deux raccords de tuyau (11, 16).

3. Dispositif selon l'une des revendications précédentes, **caractérisé en ce que** le corps tournant (1) présente un prolongement radial beaucoup plus grand que son prolongement axial.

4. Dispositif selon l'une des revendications précédentes, **caractérisé en ce que** la surface est plane et forme un angle droit avec l'axe de rotation du corps tournant (1).

5. Dispositif de filtration selon au moins une des revendications précédentes, **caractérisé en ce que** le corps tournant (1) peut être entraîné sans contact.

6. Dispositif de filtration selon la revendication 5, **caractérisé en ce que** le corps tournant (1) est métalliquement conducteur et/ou magnétisable.

7. Dispositif de filtration selon au moins une des revendications précédentes, **caractérisé en ce que** sur le côté de la membrane filtrante (2) tourné à l'opposé de la chambre de liquide, il est prévu des nervures de retenue de membrane (6) qui soutiennent la membrane (2) de telle manière que la forme d'interstice de la chambre de liquide soit conservée, même en cas de sollicitation mécanique de la membrane filtrante (2).

8. Dispositif de filtration selon au moins une des revendications précédentes, **caractérisé par** deux moitiés de boîtier (4a, 4b) qui enferment entre elles le corps tournant (1) et sont réalisées de manière à obtenir deux chambres de liquide (12) en forme d'interstice identiques de part et d'autre du corps tournant (1).
